# EUROPEAN PATENT SPECIFICATION

(11) **EP 2 398 343 B1**
(45) Date of publication and mention of the grant of the patent: **06.07.2016**
(21) Application number: 10703417.5
(22) Date of filing: 03.02.2010
(51) Int. Cl.: A23L 2/58, A23G 3/36, A23G 3/34, A23G 3/48, A23G 9/32, A23G 9/42, C09B 61/00, C09B 67/22, C09B 67/02, A23L 5/42

(54) **Food grade colouring agent**
Für Lebensmittel geeignete Färbemittel
Agent de coloration de qualité alimentaire

(30) Priority: 21.02.2009 EP 09002501; 01.10.2009 EP 09012445
(43) Date of publication of application: 28.12.2011
(73) Proprietor: Ecochroma AG, 8048 Zürich (CH)
(72) Inventor: RUGERIS, Jess Edward, West Molesey Surrey KT8 1QU (GB)
(74) Representative: Bremi, Tobias Hans
(86) International application number: PCT/EP2010/000652
(87) International publication number: WO 2010/094398

(56) References cited:
- EP-A1- 1 293 539
- WO-A1-02/087353
- WO-A1-03/010240
- DE-B- 1 114 696
- FR-A1- 2 914 311
- JP-A- 3 215 569
- JP-A- 62 003 744
- US-A- 5 958 481
- US-A1- 2003 082 281
- US-A1- 2004 022 904
- US-A1- 2006 003 060
- US-A1- 2007 218 189
- DATABASE WPI Week 198707 Thomson Scientific, London, GB; AN 1987-046412 XP002607890 & JP 62 003741 A (SAN-EI CHEM IND LTD) 9 January 1987 (1987-01-09)

## Description

The present invention relates to a method for the preparation of a food grade colouring agent, and the food grade colouring agent thus obtained. Its use for the preparation of coloured ice cubes or confectionary is also disclosed.

Nowadays, a wide range of food grade colouring agents is used by the food industry in order to enhance the optical appearance of food.

Of the known food grade colouring agents, only a few are natural, such as betanoin or chlorophyll, for instance. Most food grade colouring agents are either synthetic analogs of naturally occurring substances - so-called nature identical substances - or are fully artificial.

The use of some of the artificial and synthetic colouring agents, e.g. of the azo dyes, for colouring food is highly controversial, as they are known or suspected to be allergenic, carcinogenic, or to have other undesired side effects on the human body or the environment.

It is therefore a problem of the present invention to provide a method for preparation of food grade colouring agents in a wide range of colours, which are absolutely safe to use.

With respect to the preparation of coloured ice cubes and confectionary, for instance, it is particularly desirable not only to provide a wide range of colours, but also to avoid undesired side effects of such a colouring agent:
- It is desirable that coloured ice cubes can be used with essentially any kind of drink and coloured confectionary materials for any kind of food. Thus, in order to avoid unpleasant taste or undesired mixing of tastes, the coloured ice cubes or confectionary should be taste- and odourless.
- From a visual point of view, coloured ice cubes should not change the colour of the drink, for the cooling of which they are used.
- Since the coloured ice cubes or confectionary materials may by brought into contact with textiles, for instance if a drink is spilled on clothes, it is further desirable that the colouring agent, and thus the coloured ice cubes or confectionary, does not stain textiles, i.e. that it is "non-staining".

It is therefore a further problem of the present invention to provide food grade colouring agents which is "non-staining" and does not have a taste or smell.

US2007/0218189 describes agents which inhibit reduction of active oxygen eliminating activity, and which can be isolated from a variety of plants extracts based on plants such as such Chinese cabbages, cabbages and multiple others. The plant extract may be prepared from freshly crushed leaves of red cabbage.

JPS623744 describes red or reddish purple ice cream stable to heat, light and pH, obtained by colouring ice cream with a dry powder of a dyestuff of red cabbage.

EP1293539 discloses the extraction of fresh crushed leaves of red cabbage in order to prepare a colorant formulation.

DE1115696 discloses the stabilisation of a red coloring agent from plants such as red cabbage by ultrafiltering and drying the juice immediately after pressing without further extraction.

The problem is solved by the method according to claim 1 and the colouring agent according to claims 5. Preferred embodiments are subject of the dependent claims.

The present invention provides for a method for preparation of a food grade colouring agent, which comprises dried red cabbage.

The colouring agent obtained by the method of the present invention is very potent. As it is derived from red cabbage, i.e. a vegetable material, it is absolutely safe for use in food, drinks, and other products, which are applied to the human body, such as pharmaceutical or cosmetic products, for instance, and in particular for the preparation of coloured ice cubes or coloured confectionary. In order to obtain a particularly safe colouring agent, it is further possible to use organic red cabbage.

The colouring agent obtained by the method of the present invention has essentially no taste. In general, a tasteless substance will also be odourless. In the food industry and, in particular, in the beverage and confectionary industries, the use of colouring agents is common practice. Thanks to the colouring agent of the present invention, it is possible to conserve the original smell and taste of the food or drink. Therefore, the colouring agent of the present invention is particularly well suited for the preparation of coloured drinks, ice cubes or confectionary.

The colouring agent obtained by the method according to the present invention comprises at least dried red cabbage. Colouring agents comprising dried red cabbage offer a wide range of colours: Depending on the parameters used for the preparation of the colouring agent, such as the drying conditions, and on the particle size of the colouring agent, various shades of blue, turquoise, and purple can be obtained. In addition, it is possible to add other vegetable materials, such as dried lemon or saffron, in order to obtain an even wider range of colours, including various shades of green, orange, red, and pink.

Preferably, the colouring agent obtained by the method of the present invention does not comprise any synthetic dyes or other, non-natural additives. In a preferred embodiment, the food grade colouring agent of the present invention contains only dried red cabbage, which is preferably in granulate or powder form.

By using dried red cabbage, it is possible to prepare a very concentrated colouring agent, thus minimizing the amount of colouring agent necessary to colour a certain amount of a food product. The colour of the colouring agent of the present invention is especially stable and insensitive towards oxidation, as it is still bound by the dried red cabbage.

From a colouring agent obtained by the method of the present invention, which comprises at least dried red cabbage, the colour can easily be extracted using water or steam.

Alternatively, it is also possible to extract the colour using ethanol or an ethanol/water mixture. For instance, the colouring agent may be extracted with pure grain alcohol, such as the one used for the production of Ouzo or Sambuca. An extraction with ethanol affords a coloured liquid concentrate, which may be used as such or, alternatively, from which the ethanol may also be evaporated to afford a coloured, water soluble powder.

A further advantage of the colouring agent obtained by the method according to the present invention is that it is "non-staining": If a liquid coloured with the colouring agent is poured through a white cotton cloth, for instance, the cloth is not stained and remains white.

The colouring agent obtained by the method of the present invention comprises dried red cabbage. By adjusting the drying conditions, such as temperature, drying method or drying time, a colouring agent in a predetermined colour is obtained. A person skilled in the art is able to determine the exact conditions using pertinent preliminary tests.

In a preferred embodiment, the colouring agent obtained by the method comprises air or freeze dried red cabbage. Alternatively, the red cabbage can also be spray dried. These drying methods allow for a fast and gentle drying of the red cabbage, affording the colouring agent in the desired colour and with a high colouring strength.

Air drying is particularly favourable for drying the red cabbage, as it allows preserving the desired colours while most of the aroma is removed. In addition, the colouring agent prepared by air drying is particularly stable. Even after several months of storage under air, no oxidation is observed. Since colouring agents comprising freeze dried red cabbage are somewhat less stable, they are preferably stored under vacuum to prevent oxidation.

In a preferred embodiment, the food grade colouring agent obtained by the method of the present invention contains only dried red cabbage, which is preferably in granulate or powder form.

In a preferred embodiment of the present invention, the colouring agent obtained by the method of the present invention is in powder form. Such a powder is obtained by drying and subsequent grinding of the dried red cabbage. Due to the relatively large surface area of the individual particles, a colouring agent in powder form is particularly potent and the colour can be very easily extracted therefrom. In addition, a red cabbage based colouring agent in powder form yields a very particular range of colours.

In an alternative preferred embodiment, the colouring agent obtained by the method of the present invention is a granulate. The colouring agent granulate is obtained by chopping the red cabbage into pieces of about 2 to 4 cm before drying. Compared to the powder, the granulate is more stable towards oxidation. If the colouring agent of the present invention is provided as a granulate, it affords a particular range of colours, which is different from the one observed for the corresponding powder.

In a preferred embodiment, at least the colour of the colouring agent obtained by the method of the present invention is soluble in water and/or steam. This allows for extraction of the colour using water and/or steam immediately before the introduction into the product to be coloured. Preferably, only the colour is soluble in water and/or steam, whereas the rest of the colouring agent is not soluble and can easily be removed from the water or steam by filtration after use.

More preferably, the colour of the colouring agent obtained by the method of the present invention is also soluble in cold water, which is the case if the colouring agent of the present invention is supplied in powder form. Preferably, the colour of the colouring agent can be extracted with cold water having a temperature of less than 30 °C, more preferably of less than 20 °C, and most preferably in the range of 5 °C to 15 °C.

In a preferred embodiment of the present invention, the colouring agent obtained by the method of the present invention is in a gas and/or liquid permeable package, preferably a sachet or pod. Inserting the dried red cabbage into such a package offers a wide range of advantages: The colouring agent can be allotted in the desired amounts into each package to provide a single dose. Several single dose packages can then be further packaged into a container suitable for storage, transport and sale of the colouring agent. When using the colouring agent, it is not necessary to touch the dried red cabbage directly, but only the surrounding package, thus avoiding contamination. Furthermore, during the actual colouring process, the gas and/or liquid permeable package containing the food grade colouring agent can be inserted into a liquid or gas, which is able to extract the colour. The colour itself is "washed out" of the package by the liquid or gas, whereas the solid residues of the colouring agent are kept inside the package and can easily be removed from the extraction liquid or gas.

In a preferred embodiment, the colouring agent obtained by the method of the present invention provides a blue colour, which is preferably in the range of the Pantone colours No. 072, 285, 286, 292, 293, 297, 299, 300, 306, 631, and 801. Such a blue colouring agent is obtainable by chopping and drying red cabbage for 7 to 11 hours at about 50 °C to 55 °C in order to obtain a granulate. The blue colouring agent is very potent and affords a coloured liquid of a very brilliant blue. In addition, thanks to the method of preparation used for the colouring agent of the present invention, the colouring agent is "non-staining", whereas red cabbage itself does very well stain textiles and similar materials.

In another preferred embodiment, the colouring agent obtained by the method of the present invention provides a purple colour, which is preferably in the range of the Pantone colours No. 527 and 528. Such a purple colouring agent is by chopping and drying red cabbage for 7 to 11 hours at about 50 °C to 55 °C, followed by grinding of the dried red cabbage to obtain a powder. The purple colouring agent is very potent and affords a coloured liquid of a very brilliant purple. In addition, thanks to the method of preparation used for the colouring agent of the present invention, the colouring agent is "non-staining", whereas red cabbage itself does very well stain textiles and similar materials.

In another preferred embodiment, the colouring agent obtained by the method of the present invention provides a turquoise colour, which is preferably in the range of the Pantone colours No. 333, 335, 377, 3405, 346, and 354. A turquoise colour is obtainable by extracting about 1 to 2 g of the above described granulate with about 10 to 20 ml of hot water and applying it to sugar. Upon heating of the sugar to > 102 °C, the colour changes from sapphire blue to turquoise shades. The turquoise colouring agent is very potent and affords confectionary of a very brilliant turquoise blue or turquoise green.

Further preferred colours obtainable with the colouring agent obtained by the method of the present invention include shades of red and pink, for instance in the range of the Pantone colours No. 185 and 1925 (ruby and rhodamine red). Red and pink shades can be obtained by adding a few drops of lemon juice to the dried red cabbage.

In a further aspect, the present invention also relates to a method for the preparation of a food grade colouring agent. The food grade colouring agent is obtained by drying one or more coloured vegetable materials under conditions resulting in a predetermined colour. The one or more coloured vegetable materials are selected from the group consisting of red cabbage, beetroot, lemon, black currant, red currant, strawberry, blackberry, blueberry, cranberry, and saffron.

The method of the present invention affords a potent colouring agent, which is derived from coloured vegetable materials. It is therefore absolutely safe for use in food, drinks, and other products, which are applied to the human body, such as pharmaceutical or cosmetic products. In addition, the method of the present invention allows for a very straight forward, cost effective preparation of the colouring agent.

The coloured vegetable materials are dried under conditions resulting in a predetermined colour. By drying the vegetable materials, it is possible to prepare a very concentrated colouring agent, thus minimizing the amount of colouring agent necessary to colour a certain amount of a food product. The colour of the colouring agent of the present invention is especially stable and insensitive towards oxidation, as it is still bound by the dried vegetable material. At the same time, the method of the present invention allows for the preparation of a food grade colouring agent having a predetermined colour by adjustment of the drying conditions, such as temperature, drying method or drying time. A person skilled in the art is able to determine the exact conditions using pertinent preliminary tests.

The term "vegetable materials", as used throughout this application, includes not only entire fruits or vegetables, but also selected parts of fruits or vegetables, such as leafs or petals. The vegetable materials, which are preferably used in the method of the present invention, are particularly colourful and afford a potent colouring agent when dried. In addition, it is possible to prepare a wide range of different colours starting from one or more of the above coloured vegetable materials.

In order to obtain a particularly safe colouring agent, it is possible to use organic vegetable materials.

In a preferred embodiment, the coloured vegetable materials are air or freeze dried. Alternatively, the coloured vegetable materials can also be spray dried. These drying methods allow for a fast and gentle drying of the vegetable materials, affording the colouring agent in the desired colour and with a high colouring strength.

Air drying is particularly favourable for drying the coloured vegetable materials, as it allows preserving the desired colours while most of the aroma is removed. In addition, the colouring agent prepared by air drying is particularly stable. Even after several months of storage under air, no oxidation is observed. Freeze drying, on the other hand, allows preserving some of the flavours originally contained in the coloured vegetable materials. It is therefore possible to obtain a colouring agent from lemon zest having a slight lemon smell. Preferably, freeze dried colouring agents are stored under vacuum to prevent oxidation.

In order to facilitate the drying process, it is preferred that the coloured vegetable materials are cut or ground before being dried. In this manner, the surface area of the vegetable materials is enhanced and the drying process accelerated. The method of this preferred embodiment affords the colouring agent in relatively small particles having a large surface area, which can easily be measured and dosed. Furthermore, the enlarged surface area also improves the colouring agent's colouring properties, since the colour is released through the surface.

Alternatively, it is also possible that the coloured vegetable materials are cut or ground after the drying.

In a preferred embodiment, two or more coloured vegetable materials are mixed after having been dried. By mixing several vegetable materials, it is possible to prepare food grade colouring agents in a large variety of different colours and in various shades. Thus, the colour can be adjusted to the needs of the industry and the desires of the consumers.

Alternatively, it is also possible that two or more coloured vegetable materials are mixed prior to drying.

In a preferred embodiment of the present invention, the dried vegetable material is inserted into a gas and/or liquid permeable package, preferably a sachet or pod. Inserting the dried vegetable material into such a package offers a wider range of advantages: The colouring agent can be allotted in the desired amounts into each package to provide a single dose. Several single dose packages can then be further packaged into a container suitable for storage, transport and sale of the colouring agent. When using the colouring agent, it is not necessary to touch the dried vegetable substances directly, but only the surrounding package, thus avoiding contamination. Furthermore, during the actual colouring process, the gas and/or liquid permeable package containing the food grade colouring agent can be inserted into a liquid or gas, which is able to extract the colour. The colour itself is "washed out" of the package by the liquid or gas, whereas the solid residues of the colouring agent are kept inside the package and can easily be removed from the extraction liquid or gas.

In particular, the present invention also relates to a method for the preparation of the colouring agent of the present invention. Said method comprises the steps of chopping and drying red cabbage. The colouring agent thus obtained is generally in granulate form. Optionally, depending on the desired colour, the dried red cabbage is ground to afford the colouring agent in powder form.

The method of the present invention allows for a very straight forward, cost effective preparation of the colouring agent. The method of the present invention affords a potent colouring agent, which is derived from red cabbage. It is therefore absolutely safe for use in food, drinks, and other products, which are applied to the human body, such as pharmaceutical or cosmetic products. In order to obtain a particularly safe colouring agent, it is possible to use organic vegetable materials.

In order to facilitate the drying process, the red cabbage is cut (chopped) or ground before being dried. In this manner, the surface area of the red cabbage is enhanced and the drying process accelerated. The method of this preferred embodiment affords the colouring agent in relatively small particles having a large surface area, which can easily be measured and dosed. Furthermore, the enlarged surface area also improves the colouring agent's colouring properties, since the colour is released through the surface.

The red cabbage is dried under conditions resulting in a predetermined colour. By drying the red cabbage, it is possible to prepare a very concentrated colouring agent, thus minimizing the amount of colouring agent necessary to colour a certain amount of a food product. The colour of the colouring agent of the present invention is especially stable and insensitive towards oxidation, as it is still bound by the dried vegetable material. At the same time, the method of the present invention allows for the preparation of a food grade colouring agent having a predetermined colour by adjustment of the drying conditions, such as temperature, drying method or drying time. A person skilled in the art is able to determine the exact conditions using pertinent preliminary tests.

In a preferred embodiment, the red cabbage is air or freeze dried. Alternatively, the red cabbage can also be spray dried. These drying methods allow for a fast and gentle drying of the vegetable materials, affording the colouring agent in the desired colour and with a high colouring strength.

Air drying is particularly favourable for drying the red cabbage, as it allows preserving the desired colours while most of the aroma is removed. In addition, the colouring agent prepared by air drying is particularly stable. Even after several months of storage under air, no oxidation is observed.

By chopping the red cabbage into pieces of about 2 to 4 cm and drying the cut red cabbage, a colouring agent in granulate form is obtained. The granulate is more stable towards oxidation than a powder, and the extraction of the granulate affords a different range of colours than the extraction of the powder.

In order to obtain a colouring agent in powder form, on the other hand, the dried red cabbage is ground. Thanks to the grinding, the surface area of the particles of the colouring agent is maximized, thus facilitating the release of colour from the colouring agent. Surprisingly, it has been found that it is even possible to extract the colour from the colouring agent with cold water, if the colouring agent is in powder form, whereas hot water or steam is necessary in the case of a colouring agent in granulate form.

As has been described above, the colour range of the colouring agent varies with its particle size, the powder affording purple-blue shades and the granulate affording blue or turquoise shades.

In a preferred embodiment, the dried red cabbage is mixed with one or more other coloured vegetable materials, which have preferably also been dried. For instance, the dried red cabbage can be mixed with dried lemon zest or saffron. By mixing several dried vegetable materials, it is possible to prepare food grade colouring agents in a large variety of different colours and in various shades. Thus, the colour can be adjusted to the needs of the industry and the desires of the consumers. Alternatively, it is also possible that one or more coloured vegetable materials are mixed with the red cabbage prior to drying.

In a further aspect, the present invention also relates to the use of the food grade colouring agent for the preparation of a coloured liquid by extracting the colouring agent with water or steam. By extracting the colouring agent of the present invention with water or steam, the colour is dissolved in the water or steam and - in the case of steam, after condensation - a coloured liquid is obtained.

In particular, the present invention also features a method for the preparation of a coloured liquid, wherein the colouring agent of the present invention is extracted with water and/or steam to afford the coloured liquid. This method allows for a very simple, fast, and straightforward preparation of a coloured liquid.

In a preferred embodiment, the coloured liquid is prepared by extracting the food grade colouring agent with cold water. This is possible if the colouring agent used is in powder form. Preferably, the cold water used for extraction has a temperature of less than 30 °C, more preferably of less than 20 °C, and most preferably in the range of 5 °C to 15 °C. The use of cold water for the extraction of the food grade colouring agent of the present invention is not only advantageous from an energetic point of view, as it saves the energy necessary for heating, but it also further facilitates the preparation of an odourless and tasteless coloured liquid.

If the coloured liquid is prepared by extracting the food grade colouring agent with hot water, which is necessary in case the colouring agent is in granulate form, the water used for extraction has preferably a temperature of at least 45 °C, more preferably of at least 70 °C. At a water temperature of more than 70 °C, pathogens potentially contained in the water are killed.

Alternatively, it is also possible to use the food grade colouring agent of the present invention for colouring a non-water-based liquid.

The coloured liquid obtained by extraction of the food grade colouring agent of the present invention is preferably used for the preparation of coloured ice cubes, coloured drinks or coloured food, such as confectionary. Alternatively, it can also be used to prepare coloured pharmaceutical or cosmetic products. In all these cases, the characteristics of the colouring agent of the present invention described above - namely simple and cost effective preparation, availability of a wide range of colours in different shades, and complete safety for human and animal beings - are particularly advantageous.

In order to prepare coloured ice cubes, the coloured liquid is frozen and brought into the desired shape by the typical methods for the preparation of ice cubes commonly used. The coloured ice cubes thus obtained are very colourful and bright. Furthermore, the colouring agents of the present invention allow for the preparation of ice cubes in a wide range of colours, from green to turquoise and blue and to purple, pink, red, and orange.

For the preparation of coloured confectionary, sugar is added to the coloured liquid and heated. In order to prepare turquoise coloured confectionary, sugar is boiled with blue water. In order to prepare red or purple coloured confectionary, sugar is boiled with blue water and a few drops of lemon juice. In the later case, a syrupy confectionary material is obtained, whereas the turquoise coloured confectionary material becomes solid after cooling.

### Examples

### Drying apparatuses

The coloured vegetable materials are in general air dried.

As a simple arrangement, a 28x28x76 cm cardboard box with a drying rack in the closed area, an internal 60 W light bulb, and breathing vents on top can be used. The coloured vegetable material is placed on the drying rack and the light bulb is used to create dry heat.

Alternatively, it is also possible to use a commercial food drying machine or dehydrator, such as an "Excalibur Food Dehydrator" from Excalibur Products in Sacramento, USA. These "Excalibur Food Dehydrators" comprise a plastic box with several trays, on which the food to be dried is placed, an adjustable heater and an adjustable timer.

### Example 1: Preparation of a blue food grade colouring agent

500 g of fresh, raw red cabbage leaves were cut into pieces of about 2 to 4 cm and air dried at a drying temperature of about 52 °C over a period of 7 to 11 hours to afford 50 g of dried red cabbage in granulate form. The dried red cabbage granulate was then, in an amount of 1 g each, inserted into sachets. One sachet was used to produce about 8-9 1 of deep blue water by extraction with hot water having a temperature of 70 to 100 °C. The blue water was used for the preparation of blue ice cubes. The blue colour typically observed is in the range of the Pantone colours No. 072, 285, 286, 292, 293, 297, 299, 300, 306, 631, and 801.

### Example 2: Preparation of a green food grade colouring agent

1 g of dried red cabbage in granulate form, which was obtained by the method described in example 1, was mixed in a ratio of 5:1 with dried saffron. The mixture was dipped into hot water having a temperature of 70 °C for 1 second and then allowed to air dry at a temperature of 35 °C over a period of 24 hours. The dried mixture was inserted into a sachet, which was used to produce up to 9 1 of lime green water by extraction with hot water having a temperature of 70 to 100 °C. The lime green water was used for the preparation of lime green ice cubes. The blue colour typically observed is in the range of the Pantone colours No. 368 and 375.

### Example 3: Preparation of a pink food grade colouring agent

1 g of dried red cabbage granulate, which was obtained by the method described in example 1, was soaked with 5 ml of lemon juice and was then allowed to air dry at a temperature of 35 °C over a period of 24 hours. The dried mixture was, in an amount of 1 g each, inserted into sachets. One sachet was used to produce up to 9 1 of pink water by extraction with hot water having a temperature of 70 to 100 °C. The pink water was used for the preparation of pink ice cubes. The pink colour typically observed is in the range of the Pantone colours No. 185 and 1925.

### Example 4: Preparation of a purple food grade colouring agent

100 g of cooked or raw beetroot was air dried at a drying temperature of 35 °C over a period of 4 days to afford 15 g of dried beetroot. The dried beetroot was granulated.

0.5 g of dried and granulated red cabbage, which was obtained by the method described in example 1, was mixed with 0.5 g of dried and granulated beetroot. The mixture was inserted into a sachet, which was used to produce up to 9 1 of purple water by extraction with hot water having a temperature of 70 to 100 °C. The purple water was used for the preparation of purple ice-cubes.

In this context, the use of cooked beetroot proved advantageous, as the derived purple ice-cubes were less tainting upon melting.

### Example 5: Preparation of an orange food grade colouring agent

1 g of dried red cabbage in granulate form, which was obtained by the method described in example 1, was mixed in a ratio of 5:1 with dried saffron. 4 to 5 drops of lemon juice were added to the mixture and it was then allowed to air dry. The dried mixture was granulated and, in an amount of 1 g each, inserted into sachets. Each sachet can be used to produce up to 7 1 of orange or gold coloured water by extraction with hot water having a temperature of 70 to 100 °C. The orange water was used for the preparation of orange ice cubes.

### Example 6: Preparation of a turquoise food grade colouring agent

1 g of dried red cabbage, which was obtained by the method described in example 1, was mixed in a ratio of 5:1 with dried saffron. The mixture was granulated and inserted into a sachet, which can be used to produce about 1.5 to 2 1 of turquoise water by extraction with hot water having a temperature of 70 to 100 °C. The turquoise water was used for the preparation of turquoise ice-cubes.

### Example 7: Preparation of a yellow food grade colouring agent having a lemon smell

30 g of lemon zest, which was obtained from mature lemons having a dark yellow colour, was air dried using the drying apparatus described above at a temperature of 35 °C over a period of 24 hours to afford 3 g of dried lemon zest. The dried lemon zest was granulated and, in an amount of 1 g each, inserted into sachets. One sachet was used to produce about 1.5 to 2 1 of yellow water having a lemon smell. The yellow water was used for the preparation of yellow ice-cubes.

### Example 8: Preparation of a blue food grade colouring agent for use in confectionary

For a blue food grade colouring agent to be used in confectionary, red cabbage is air dried at a drying temperature of about 68 °C over a period of about 12 hours. The blue colouring agent thus obtained shows improved heat stability and can therefore be used for the manufacture of confectionary at elevated temperatures without a significant change in colour.

### Example 9: Preparation of a turquoise confectionary material

The blue water obtained in example 1 was added to sugar and heated to > 100 °C to afford a turquoise confectionary material, which upon cooling becomes solid. The turquoise colour typically observed is in the range of the Pantone colours No. 333, 335, 377, 3405, 346, and 354.

### Example 10: Preparation of a red or pink confectionary

### material

The blue water obtained in example 1 and a few drops of lemon juice was added to sugar and heated to > 100 °C to afford a confectionary material in shades of red or pink, depending on the amount of lemon juice added. Upon cooling, the red or pink confectionary material stays liquid and adopts a syrupy texture. The red or pink colour typically observed is in the range of the Pantone colours No. 185 and 1925.

### Example 11: Preparation of a blue confectionary material for hard boiled sweets

The blue colouring agent obtained in example 8 was added to isomalt and heated to > 145 °C to afford a blue confectionary material.

### Further Colours

In examples 1, 2, and 6, the preparation of a blue, green, and turquoise food grade colouring agent has been described, respectively. By altering the ratio of red cabbage to saffron, as will be well understood by a person skilled in the art, various shades of green, turquoise, and blue colouring agents can be obtained.

In examples 2, 5, and 6, the preparation of a green, orange, and turquoise food grade colouring agent has been described, respectively. By replacing saffron with dried lemon zest, food grade colouring agents of similar colours but with a lemon smell can be obtained.

## Claims

1. Method for the preparation of a food grade colouring agent, said method consisting of chopping and drying red cabbage, and optionally grinding the dried red cabbage after the drying.

2. Method according to claim 1, wherein the red cabbage is air dried.

3. Method according to claim 1, wherein the red cabbage is freeze dried.

4. Food grade colouring agent obtained by the method according to one of claims 1 to 3.

5. Colouring agent according to claim 4, wherein the colouring agent contains only dried red cabbage.

6. Colouring agent according to claim 4 or 5, wherein the colouring agent is in powder form.

7. Colouring agent according to one of claims 4 to 6, wherein the colouring agent is a granulate.

8. Colouring agent according to one of claims 4 to 7, wherein at least the colour of the colouring agent is soluble in water and/or steam, preferably in cold water.

9. Colouring agent according to one of claims 4 to 8, wherein the colouring agent is in a gas and/or liquid permeable package, preferably in a sachet or pod.

10. Colouring agent according to one of claims 4 to 9, wherein the colouring agent provides a blue colour, which is preferably in the range of the Pantone colours No. 072, 285, 286, 292, 293, 297, 299, 300, 306, 631, and 801.

11. Colouring agent according to one of claims 4 to 10, wherein the colouring agent provides a purple colour, which is preferably in the range of the Pantone colours No. 527 and 528.

12. Colouring agent according to one of claims 4 to 11, wherein the colouring agent provides a turquoise colour, which is preferably in the range of the Pantone colours No. 333, 335, 377, 3405, 346, and 354.

## Patentansprüche

1. Verfahren zur Herstellung eines für Lebensmittel geeigneten Färbemittels, wobei das besagte Verfahren aus Zerkleinern und Trocknen von Rotkohl, und optional aus Mahlen des getrockneten Rotkohls nach dem Trocknen, besteht.

2. Verfahren gemäss Anspruch 1, wobei der Rotkohl luftgetrocknet ist.

3. Verfahren nach Anspruch 1, wobei der Rotkohl gefriergetrocknet ist.

4. Für Lebensmittel geeignetes Färbemittel, welches durch das Verfahren gemäss einem der Ansprüche 1 bis 3 erhalten wird.

5. Färbemittel gemäss Anspruch 4, wobei das Färbemittel nur getrockneten Rotkohl enthält.

6. Färbemittel gemäss Anspruch 4 oder 5, wobei das Färbemittel pulverförmig ist.

7. Färbemittel gemäss einem der Ansprüche 4 bis 6, wobei das Färbemittel ein Granulat ist.

8. Färbemittel gemäss einem der Ansprüche 4 bis 7, wobei zumindest die Farbe des Färbemittels in Wasser und/oder Dampf, vorzugsweise in kaltem Wasser, löslich ist.

9. Färbemittel gemäss einem der Ansprüche 4 bis 8, wobei das Färbemittel in einer gas- und/oder flüssigkeitsdurchlässigen Verpackung, vorzugsweise in einem Beutel oder Hülse, ist.

10. Färbemittel gemäss einem der Ansprüche 4 bis 9, wobei das Färbemittel eine blaue Farbe liefert, welche vorzugsweise im Bereich der Pantone-Farben Nr. 072, 285, 286, 292, 293, 297, 299, 300, 306, 631 und 801 ist.

11. Färbemittel gemäss einem der Ansprüche 4 bis 10, wobei das Färbemittel eine violette Farbe liefert, welche vorzugsweise im Bereich der Pantone-Farben Nr. 527 und 528 ist.

12. Färbemittel gemäss einem der Ansprüche 4 bis 11, wobei das Färbemittel eine türkisblaue Farbe liefert, welche vorzugsweise im Bereich der Pantone-Farben Nr. 333, 335, 377, 3405, 346 und 354 ist.

## Revendications

1. Procédé pour la préparation d'un agent colorant de qualité alimentaire, ledit procédé consistant à hacher et sécher du chou rouge, et optionnellement moudre le chou rouge séché après le séchage.

2. Procédé selon la revendication 1, dans lequel le chou rouge est séché à l'air.

3. Procédé selon la revendication 1, dans lequel le chou rouge est séché par lyophilisation.

4. Agent colorant de qualité alimentaire obtenu par le procédé d'une des revendications 1 à 3.

5. Agent colorant selon la revendication 4, dans lequel l'agent colorant contient uniquement du chou rouge séché.

6. Agent colorant selon les revendications 4 ou 5, dans lequel l'agent colorant est en forme de poudre.

7. Agent colorant selon une des revendications 4 à 6, dans lequel l'agent colorant est un granulé.

8. Agent colorant selon une des revendications 4 à 7, dans lequel au moins la couleur de l'agent colorant est soluble dans de l'eau et/ou de la vapeur, préférablement dans de l'eau froide.

9. Agent colorant selon une des revendications 4 à 8, dans lequel l'agent est dans une enveloppe perméable au liquide et/ou gaz, préférablement dans un sachet ou gousse.

10. Agent colorant selon une des revendications 4 à 9, dans lequel l'agent colorant fournit une couleur bleue, qui est préférablement dans le domaine des couleurs Pantone no. 072, 285, 286, 292, 293, 297, 299, 300, 306, 631 et 801.

11. Agent colorant selon une des revendications 4 à 10, dans lequel l'agent colorant fournit une couleur mauve, qui est préférablement dans le domaine des couleurs Pantone no. 527 et 528.

12. Agent colorant selon une des revendications 4 à 11, dans lequel l'agent colorant fournit une couleur turquoise, qui est préférablement dans le domaine des couleurs Pantone no. 333, 335, 377, 3405, 346 et 354.
